# FASCICULE DE BREVET EUROPEEN

(11) **EP 0 539 484 B1**
(45) Date de publication et mention de la délivrance du brevet: **15.01.1997**
(21) Numéro de dépôt: 91913608.5
(22) Date de dépôt: 15.07.1991
(51) Int. Cl.: C07D 498/18, A61K 38/00, A61K 31/42, C07K 5/06

(54) **PROCEDE DE PREPARATION DE SULFONYLPRISTINAMYCINE IIB**
VERFAHREN ZUR HERSTELLUNG VON SULFONYLPRISTINAMYCIN IIB
METHOD FOR PREPARING SULPHONYLPRISTINAMYCIN IIB

(30) Priorité: 16.07.1990 FR 9009033
(43) Date de publication de la demande: 05.05.1993
(73) Titulaire: RHONE-POULENC RORER S.A., 92160 Antony (FR)
(72) Inventeur: RADISSON, Xavier, F-69006 Lyon (FR)
(74) Mandataire: Savina, Jacques
(86) Numéro de dépôt international: FR9100579
(87) Numéro de publication internationale: WO9201692

(56) Documents cités:
- EP-A- 0 252 720
- EP-A- 0 322 800
- FR-A- 2 576 022
- GB-A- 2 206 577

## Description

Les (dialcoylamino-2 alcoyl) sulfonyl-26 pristinamycine II_{B} de formule générale : 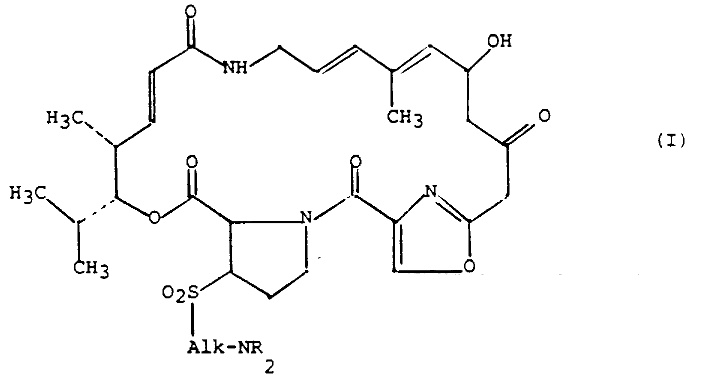 dans laquelle Alk représente un radical alcoylène droit ou ramifié et R représente des radicaux alcoyle droits ou ramifiés, ces radicaux contenant 1 à 10 atomes de carbone, sont des produits connus pour leur activité antibactérienne et leur action synergisante de l'activité antibactérienne de la pristinamycine I_{A} comme cela a été décrit dans le brevet européen 191 662.

Les dérivés de la pristinamycine II_{B} de formule générale (I) peuvent être obtenus par oxydation du sulfure correspondant notamment selon l'enseignement du brevet européen 191 662 qui décrit l'oxydation d'un sulfure de formule générale : 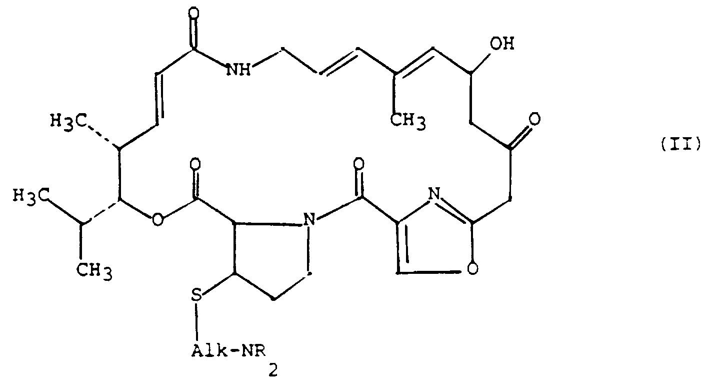 dans laquelle Alk et R sont définis comme précédemment, par l'eau oxygénée en présence de dioxyde de sélénium. La réaction s'effectue en milieu aqueux ou organique notamment dans un alcool...

Selon la demande de brevet anglais 2 206 577, les dérivés de la pristinamycine II_{B} de formule générale (I) peuvent être également obtenus par oxydation du sulfure correspondant de formule générale (II), par l'eau oxygénée en présence de tungstate de métal alcalin comme le tungstate de sodium, en milieu aqueux par exemple acétone-eau ou acétonitrile-eau, ou dans un solvant non miscible à l'eau comme un hydrocarbure chloré, à une température allant de -5°C à la température ambiante.

Il a maintenant été trouvé que la réaction d'oxydation du sulfure de formule générale (II) conduit à différents produits d'oxydation selon les conditions employées. Il a en effet été mis en évidence que l'on peut obtenir la sulfone de pristinamycine II_{B} de formule générale (I) avec des rendements considérablement améliorés, en opérant par oxydation du sulfure de pristinamycine II_{B} par 3,5 à 20 équivalents d'eau oxygénée, en présence d'un tungstate de métal alcalin comme par exemple le tungstate de sodium, en milieu biphasique, à une température comprise entre 10 et 25°C, étant entendu que la quantité d'eau introduite dans le milieu est supérieure à la limite de solubilité dans le solvant organique.

Il est nécessaire que l'oxydant soit introduit en large excès par rapport à la quantité de produit à oxyder et pour cela, que la proportion oxydant-cooxydant soit maintenue dans une certaine limite. L'eau oxygénée est employée à raison de 3,5 à 20 équivalents par mole de sulfure de pristinamycine II_{B}, la proportion de tungstate de sodium varie généralement de 5 à 0,5 %.

Le milieu biphasique est constitué d'un mélange solvant chloré-eau comme, par exemple, le mélange chlorure de méthylène-eau ou le mélange dichloroéthane-eau ou le mélange chloroforme-eau.

Il peut être également constitué d'un mélange eau-alcool non miscible à l'eau comme par exemple le mélange n.butanol-eau.

On opère avantageusement dans un mélange eau-solvant chloré 50-50 en volumes, mais il est également possible de faire varier ces proportions.

Le catalyseur préféré est le tungstate de sodium, mais il est bien entendu que la réaction peut aussi être mise en oeuvre en présence de tungstate de potassium.

Il en résulte de cette nouvelle mise en oeuvre-du procédé d'oxydation de la (dialcoylamino-2 alcoyl) thio-26 pristinamycine II_{B} en (dialcoylamino-2 alcoyl) sulfonyl-26 pristinamycine II_{B}, une amélioration très importante des rendements, du fait de la catalyse en milieu hétérogène, en présence de quantités relatives prédéterminées oxydant-cooxydant. Selon le nouveau procédé, des rendements supérieurs à 70 % peuvent être atteints.

Les exemples suivants, donnés à titre non limitatif, illustrent la présente invention.

### EXEMPLE 1

A une solution de 1 g (1,51 mM) de (diéthylamino-2 éthyl) thio-26 pristinamycine II_{B} dans 10 cm³ de chlorure de méthylène et 10 cm³ d'eau, est additionnée en une minute à la température ambiante, une solution de 5 mg (0,0151 mM, 1 % molaire) de tungstate de sodium dans l'eau oxygénée (30 %, 1,71 g, 15,1 mM, 10 équivalents). La réaction est légèrement exothermique au début. Le mélange réactionnel est agité pendant 16 heures à température ambiante.

Un dosage du mélange réactionnel met en évidence les taux de transformation et rendement suivants :
- Taux de transformation = 100 %
- Rendement réel en sulfone = 79 %

La phase organique est décantée et lavée une fois avec 20 cm³ d'eau, puis séchée sur sulfate de sodium et concentrée à sec. On obtient ainsi la (diéthylamino-2 éthyl) sulfonyl-26 pristinamycine II_{B} avec un titre de 78 % (Rendement réel = 75 %).

La (diéthylamino-2 éthyl) thio-26 pristinamycine II_{B} peut être préparée comme décrit dans le brevet EP 135 410.

### EXEMPLE 2

En opérant de la même manière qu'à l'exemple 1, mais en remplaçant le chlorure de méthylène par 10 cm³ de dichloro-1,2 éthane et en agitant le mélange réactionnel pendant 4 heures et 30 minutes, on obtient la (diéthylamino-2 éthyl) sulfonyl-26 pristinamycine II_{B} avec un titre de 71 % (Rendement réel = 70 %).

### EXEMPLE 3

A une solution de 52,966 g (75,88 mM) de (diéthylamino-2 éthyl) thio-26 pristinamycine II_{B} dans 500 cm³ de dichloro-1,2 éthane et 427 cm³ d'eau, sont additionnés successivement, 1,25 g de tungstate de sodium (3,789 mM ; 5 % molaire), puis en 2 heures et 30 minutes et à une température de 19-20°C, 27,124 cm³ (265 mM ; 3,5 équivalents) d'une solution d'eau oxygénée (30 %) dans 73 cm³ d'eau. Après agitation du mélange réactionnel pendant au total 8 heures et 40 minutes, on obtient :
- Taux de transformation = 100 %
- Rendement réel en (diéthylamino-2 éthyl) sulfonyl-26 pristinamycine II_{B} = 78 %

La phase aqueuse est décantée et extraite par 2 fois 100 cm³ de dichloro-1,2 éthane. Les phases organiques sont réunies et lavées par 3 fois 100 cm³ d'eau (jusqu'à obtention d'une eau de lavage non oxydante) puis séchées sur sulfate de sodium. Après filtration et concentration à sec sous pression réduite, on obtient 52,91 g d'un solide blanc crème (rendement pondéral = 100,9 %) de titre 74,3 % en (diéthylamino-2 éthyl) sulfonyl-26 pristina- mycine II_{B} soit un rendement réel de 75 %.

La sulfone ainsi obtenue peut être transformée en di-p.toluoyltartrate par salification par l'acide di-p-toluoyltartrique. On obtient à partir de cette sulfone brute un sel titrant 100 % avec un rendement de 82,6 %. On obtient ainsi un rendement total de 62 % pour l'oxydation suivie de la salification.

### EXEMPLE 4

En opérant de la même manière qu'à l'exemple 3, l'oxydation de 1 g (1,51mM) de (diéthylamino-2 éthyl) thio-26 pristinamycine II_{B} est réalisée dans 4 cm³ de dichloro-1,2 éthane et 12 cm³ d'eau. Après 5 heures d'agitation, on obtient la (diéthylamino-2 éthyl) sulfonyl-26 pristinamycine II_{B} avec un rendement réel de 71 %.

### EXEMPLE 5

En opérant comme à l'exemple 3, l'oxydation de 1 g (1,51 mM) de (diéthylamino-2 éthyl) thio-26 pristinamycine II_{B}, dans 16 cm³ de dichloro-1,2 éthane et 4 cm³ d'eau, produit, après 2 heures d'agitation, la (diéthylamino-2 éthyl) sulfonyl-26 pristinamycine II_{B} avec un rendement réel de 70 %.

### EXEMPLE 6

En opérant comme à l'exemple 3, l'oxydation de 1 g (1,51 mM) de (diéthylamino-2 éthyl) thio-26 pristinamycine II_{B}, dans 10 cm³ de n-butanol et 10 cm³ d'eau, produit, après 6 heures d'agitation, la (diéthylamino-2 éthyl) sulfonyl-26 pristinamycine II_{B} avec un rendement réel de 40 %.

### EXEMPLE 7

En opérant comme à l'exemple 3, 5 g (7,58 mM) de (diéthylamino-2 éthyl) thio-26 pristinamycine II_{B} sont oxydés dans 50 cm³ d'eau et 50 cm³ de dichloro-1,2 éthane par 25 mg (0,0758 mM 1 % molaire) de tungstate de sodium dihydraté et 8,59 g (7,58 mM, 10 éq.) d'eau oxygénée à 30 % en 4 heures 30 minutes entre 19 et 23°C. Après traitement, on obtient 5,18 g de (diéthylamino-2 éthyl) sulfonyl-26 pristinamycine II_{B} titrant 74 %, soit un rendement réel de 73 %.

### EXEMPLE 8

En opérant comme à l'exemple 3, 1 g (1,51 mM) de (diéthylamino-2 éthyl) thio-26 pristinamycine II_{B}, est oxydé par 5 mg (0,015 mM, 1 % molaire) de tungstate de sodium dihydraté et 0,855 g (7,55 mM, 5 éq.) d'eau oxygénée à 30 % en 8 heures entre 20 et 24°C pour produire la sulfone correspondante avec un rendement de 77 %.

### EXEMPLE 9

En opérant comme à l'exemple 8, 1 g (1,51 mM) de (diéthylamino-2 éthyl) thio-26 pristinamycine II_{B}, est oxydé en 1 heure 30 minutes à 20°C, par 25 mg (0,0755 mM, 5 % molaire) de tungstate de sodium dihydraté et 1,71 g (15,1 mM, 10 éq.) d'eau oxygénée à 30 % en (diéthylamino-2 éthyl) sulfonyl-26 pristinamycine II_{B} avec un rendement de 75 %.

### EXEMPLE 10

En opérant comme à l'exemple 8, l'oxydation de la (diéthylamino-2 éthyl) thio-26 pristinamycine II_{B} mise en oeuvre à 20°C en présence de 2,5 mg (0,0075 mM, 0,5 % molaire) de tungstate de sodium et 1,71 g (1,51 mM, 10 éq.) d'eau oxygénée à 30 %, produit, en 6 heures, la (diéthylamino-2 éthyl) sulfonyl-26 pristinamycine II_{B} avec 69 % de rendement.

### EXEMPLE 11

En opérant comme à l'exemple 10, l'oxydation de la (diéthylamino-2 éthyl) thio-26 pristinamycine II_{B} conduite avec 3,42 g (30,2 mM, 20 éq.) d'eau oxygénée à 30 % produit, en 6 heures, la (diéthylamino-2 éthyl) sulfonyl-26 pristinamycine II_{B} avec 66 % de rendement.

### EXEMPLE 12

En opérant comme à l'exemple 7 sur 1 g (1,51 mM) de (diéthylamino-2 éthyl) thio-26 pristinamycine II_{B} dans 10 cm³ de chloroforme au lieu du dichloro-1,2 éthane, on obtient la (diéthylamino-2 éthyl) sulfonyl-26 pristinamycine II_{B} avec 72 % de rendement en 15 heures.

## Revendications

1. Un procédé de préparation de (dialcoylamino-2 alcoyl) sulfonyl-26 pristinamycine II_{B} de formule générale : 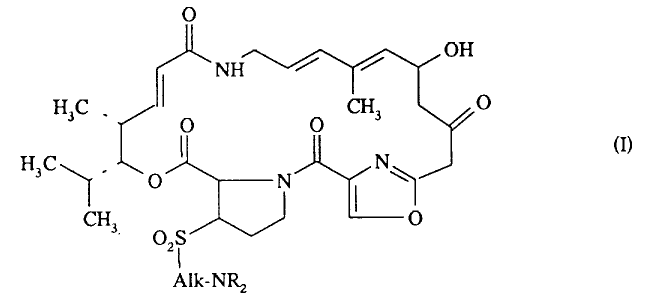 dans laquelle Alk représente un radical alcoylène droit ou ramifié et R représente des radicaux alcoyle droits ou ramifiés, ces radicaux contenant 1 à 10 atomes de carbone, par oxydation de (dialcoylamino-2 alcoyl) thio-26 pristinamycine II_{B} de formule générale : 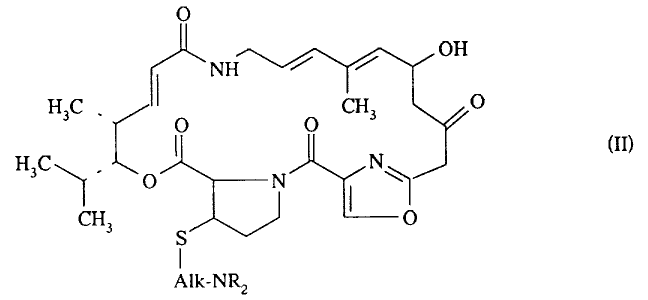 dans laquelle Alk et R sont définis comme ci-dessus, par 3,5 à 20 équivalents d'eau oxygénée en présence d'un tungstate de métal alcalin, en milieu biphasique, à une température comprise entre 10 et 25°C, étant entendu que la quantité d'eau introduite dans le milieu est supérieure à la limite de solubilité dans le solvant organique.

2. Un procédé de préparation selon la revendication 1 caractérisé en ce que le milieu biphasique comprend des rapports eau/solvant organique de 20/80 à 75/25 en volumes.

3. Un procédé selon l'une des revendications 1 ou 2 caractérisé en ce que le tungstate de métal alcalin est le tungstate de sodium.

4. Un procédé selon l'une des revendications 1 ou 2 caractérisé en ce que le tungstate de métal alcalin est introduit à raison de 5 à 0,5 % molaire.

5. Un procédé selon l'une des revendications 1 ou 2 caractérisé en ce que le milieu biphasique est constitué d'un mélange eau-solvant chloré ou eau-n.butanol.

6. Un procédé selon la revendication 5 caractérisé en ce que le mélange eau-solvant chloré est un mélange eau-chloroforme.

7. Un procédé selon la revendication 5 caractérisé en ce que le mélange eau-solvant chloré est un mélange eau-dichloroéthane.

8. Un procédé selon la revendication 5 caractérisé en ce que le mélange eau-solvant chloré est un mélange eau-dichlorométhane.

## Patentansprüche

1. Verfahren zur Herstellung von (2-Dialkylaminoalkyl)-26-sulfonyl-pristinamycin II_{B} der allgemeinen Formel 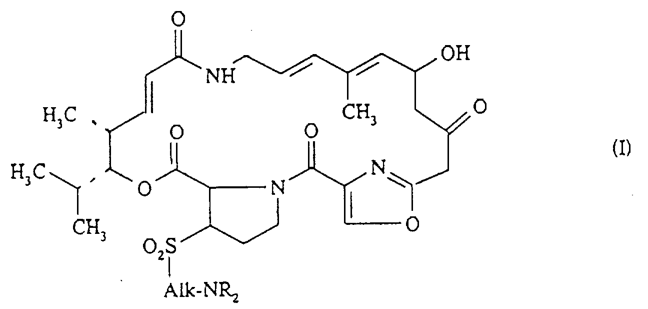 worin Alk für einen geradkettigen oder verzweigten Alkylenrest steht und R einen geradkettigen oder verzweigten Alkylrest bedeutet, wobei diese Reste 1 bis 10 Kohlenstoffatome enthalten, durch Oxidation von (2-Dialkylaminoalkyl)-26-thio-pristinamycin II_{B} der allgemeinen Formel 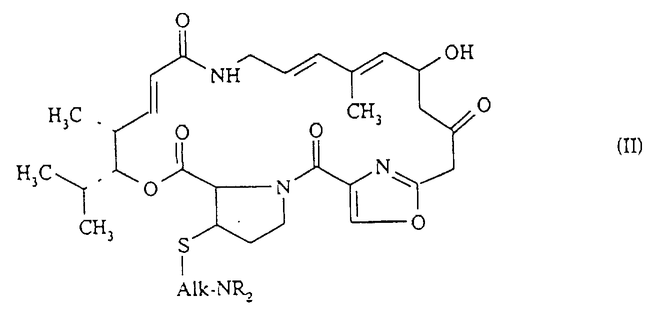 worin Alk und R wie vorstehend definiert sind, mit 3,5 bis 20 Äquivalenten Wasserstoffperoxid in Anwesenheit von Alkalimetallwolframat in einem Zwei-Phasen-Milieu bei einer Temperatur zwischen 10 und 25°C, mit der Maßgabe, daß die in das Milieu eingebrachte Menge an Wasser höher ist als die Löslichkeitsgrenze in dem organischen Lösungsmittel.

2. Herstellungsverfahren gemäß Anspruch 1, dadurch gekennzeichnet, daß das Zwei-Phasen-Milieu Verhältnisse von Wasser/organisches Lösungsmittel von 20/80 bis 75/25, ausgedrückt in Volumen, umfaßt.

3. Verfahren gemäß einem der Ansprüche 1 oder 2, dadurch gekennzeichnet, daß das Alkalimetallwolframat Natriumwolframat ist.

4. Verfahren gemäß einem der Ansprüche 1 oder 2, dadurch gekennzeichnet, daß das Alkalimetallwolframat in einer Menge von 5 bis 0,5 Mol-% eingebracht wird.

5. Verfahren gemäß einem der Ansprüche 1 oder 2, dadurch gekennzeichnet, daß das Zwei-Phasen-Milieu aus einem Gemisch von Wasser-chloriertem Lösungsmittel oder Wasser-n-Butanol besteht.

6. Verfahren gemäß Anspruch 5, dadurch gekennzeichnet, daß das Wasser-chloriertes Lösungsmittel-Gemisch ein Wasser-Chloroform-Gemisch ist.

7. Verfahren gemäß Anspruch 5, dadurch gekennzeichnet, daß das Wasser-chloriertes Lösungsmittel-Gemisch ein Wasser-Dichlorethan-Gemisch ist.

8. Verfahren gemäß Anspruch 5, dadurch gekennzeichnet, daß das Wasser-chloriertes Lösungsmittel-Gemisch ein Wasser-Methylenchlorid-Gemisch ist.

## Claims

1. Process for preparing 26-[(2-dialkylaminoalkyl)sulphonyl]pristinamycin II_{B} of general formula: 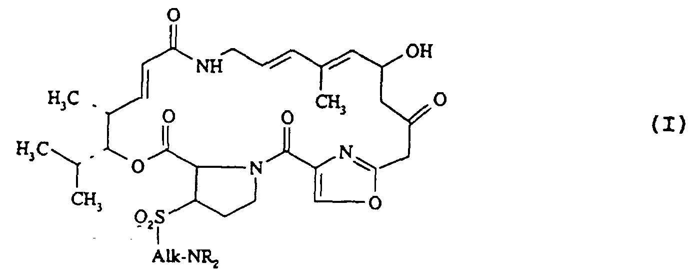 in which Alk represents a linear or branched alkylene radical and R represents linear or branched alkyl radicals, these radicals containing 1 to 10 carbon atoms, by oxidation of 26-[(2-dialkylaminoalkyl)thio]pristinamycin II_{B} of general formula: 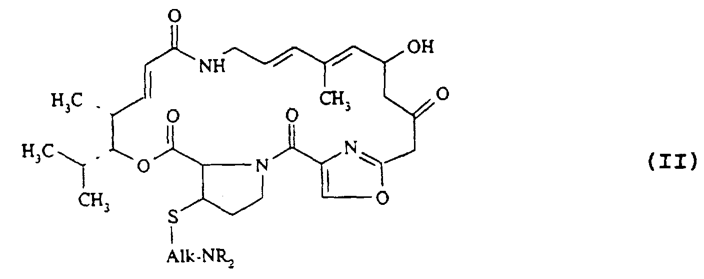 in which Alk and R are defined as above, with 3.5 to 20 equivalents of hydrogen peroxide in the presence of an alkali metal tungstate, in a two-phase medium, at a temperature of between 10 and 25°C, on the understanding that the quantity of water introduced into the medium is greater than the solubility limit in the organic solvent.

2. Preparation process according to claim 1, characterized in that the two-phase medium comprises water/organic solvent ratios of 20:80 to 75:25 by volume.

3. Process according to either of claims 1 and 2, characterized in that the alkali metal tungstate is sodium tungstate.

4. Process according to either of claims 1 and 2, characterized in that the alkali metal tungstate is introduced in the proportion of 5 to 0.5 mol%.

5. Process according to either of claims 1 and 2, characterized in that the two-phase medium consists of a water/chlorinated solvent or water/n-butanol mixture.

6. Process according to claim 5, characterized in that the water/chlorinated solvent mixture is a water/chloroform mixture.

7. Process according to claim 5, characterized in that the water/chlorinated solvent mixture is a water/dichloroethane mixture.

8. Process according to claim 5, characterized in that the water/chlorinated solvent mixture is a water/dichloromethane mixture.
